# EUROPEAN PATENT SPECIFICATION

(11) **EP 3 102 275 B1**
(45) Date of publication and mention of the grant of the patent: **01.01.2020**
(21) Application number: 15765915.2
(22) Date of filing: 20.03.2015
(51) Int. Cl.: A61M 29/00, A61F 5/08

(54) **NASAL DILATOR**
NASALER DILATATOR
DILATATEUR NASAL

(30) Priority: 21.03.2014 US 201461968798 P
(43) Date of publication of application: 14.12.2016
(73) Proprietor: NASAL MEDICAL LIMITED, Dublin 8 (IE)
(72) Inventor: O'CONNELL, Martin, Tralee, Co. Kerry 10027 (IE); YEAGER, Keith, Jersey City, NJ 07306 (US)
(74) Representative: Hanna Moore + Curley
(86) International application number: PCT/US2015/021787
(87) International publication number: WO 2015/143344

(56) References cited:
- NL-C2- 1 010 730
- US-A- 4 201 217
- US-A1- 2010 030 252
- US-A1- 2010 030 252
- US-A1- 2011 125 091
- US-A1- 2011 125 091

## Description

The current application claims a priority to the U.S. Provisional Patent application serial number 61/968,798 filed on March 21, 2014.

### FIELD OF THE INVENTION

The present invention relates to a nasal apparatus as described in the claims which improves respiration by expanding the nasal airways and potentially providing a filter to block irritating or harmful particles.

### BACKGROUND OF THE INVENTION

Respiratory issues result from a number of conditions, ranging from comparatively simple ones like congestion to more difficult ones like sleep apnea. To address these concerns, a number of products have been developed. Generally, these products are chemical based (e.g. sprays and medicine) or structural based (dilators and filters); the present invention concerns itself with the latter grouping. Placed in a person's nostrils, such devices improve breathing by expanding the nasal airways and filtering out foreign bodies which normally irritate and agitate a person's nose. The present invention improves upon these basic principles by providing an apparatus which can be used with or without a filter. The filter can be readily incorporated into the design via insert-molding, adhesive, or welding (thermal or ultrasonic). Furthermore, the apparatus is adaptable to different nostril sizes unlike many existing products which must be sold in different sizes to accommodate individual differences.

The present invention has an anatomic design influenced by 3D morphology. Existing products are simple geometries or only based on the 2D profile of the nasal opening, and are often closed profile which limits their ability to conform to a wide range of nostril sizes. The present invention is modeled on the 3D surfaces of the nasal geometry and incorporates features for maintaining a comfortable fit for a wide range of size variation from one user to the next. The current drawings show a device suitable for Type I & II nostril types, and alternate orientations of the device can be manufactured with proportions suitable for Type III & V, for Type VI & VII, and for Type IV.

Greater range of nostril size variation within a Type set, for example set I & II, is achieved by incorporating individual arms which allow flexibility in key anatomic locations. The length of the arms and the nature of the open profile design allow the device to conform to the individual users nostril. The profile cross section of the arm (generally oval shaped) is designed to allow bending along its thin dimension, while maintaining a relatively larger area of contact in its long dimension. The larger area of contact, thin bending profile, and long beam length reduce the pressure applied by the device when inserted into a relatively smaller nostril.

The profile is anatomically influenced, and has three arms. They merge on the medial side of the nostril and form the largest contact area at the septal cartilage, which is relatively planar. One arm extends in the anterior direction, following the structure of the majar alar cartilage. A second arm extends in the posterior direction and follows the curvature of the alar fibrofatty tisse structure. A third arm extends superior and arcs around to an inferior orientation, terminating near the posterior portion of the majar alar cartilage. The three arms serve to dilate the nasal cavity by acting on these structures. The flexibility of the device within and between each arm is primarily in the medial/lateral direction, and secondarily in the anterior/posterior direction. While one configuration of the present invention is described with three arms, a fourth arm may also be included for additional locational stability

The present invention acts to reduce respiratory exposure to viruses, allergens, germs, flu's, colds, bacteria, molds, dust, pet dander, pollen, pollutants, contaminants, second hand smoke, carcinogens, and other airborne contaminants. The present invention also acts to improve sleeping by increasing nasal airflow and mitigating or eliminating snoring, headaches, and nausea. An additional benefit of the present invention is increased oxygen intake and nitric oxide production, desirable to persons who want to enhance their athletic performance.

The relevant state of the art is represented by US 2011 /125091 A1, US 2010/030252 A1 and NL 1010730 C2.

### BRIEF DESCRIPTION OF THE DRAWINGS

FIG. **1** is a perspective view of an insert of the present disclosure.
FIG. **2** is a front view of an insert of the present disclosure.
FIG. **3** is a right view of an insert of the present disclosure.
FIG. **4** is a top view of an insert of the present disclosure.
FIG. **5** is a perspective view of an alternative insert configuration of the present disclosure.
FIG. **6** is a front view of an alternative insert configuration of the present disclosure.
FIG. **7** is a right view of an alternative insert configuration of the present disclosure.
FIG. **8** is a top view of an alternative insert configuration of the present disclosure.
FIG. **9** is a front view showing a compression process of the insert once positioned in the nostril.
FIG. **10** is a right view showing a compression process of the insert once positioned in the nostril.
FIG. **11** is a top view showing a compression process of the insert once positioned in the nostril.
FIG. **12** is a perspective view showing a pair of inserts attached by a bridge of the present disclosure.
FIG. **13** is a perspective view showing a filter attached to the insert of the present disclosure.
FIG. **14** is another perspective view showing a filter attached to the insert of the present disclosure.
FIG. **15** is a bottom perspective view showing a filter attached to the insert of the present disclosure.
FIG. **16** is a top view showing a filter attached to the insert of the present disclosure.
FIG. **17** is a perspective view indicating how a bridge would attach two inserts, including filters, of the present disclosure.
FIG. **18** is a perspective view of an alternative embodiment of the insert of the present disclosure.
FIG. **19** is a rear perspective view of an alternative embodiment of the insert of the present disclosure.
FIG. **20** is a rear view of an alternative embodiment of the insert of the present disclosure.
FIG. **21** is a left view of an alternative embodiment of the insert of the present disclosure.
FIG. **22** is a front view of an alternative embodiment of the insert of the present disclosure.
FIG. **23** is a top view of an alternative embodiment of the insert of the present disclosure.
FIG. **24** is a right view of an alternative embodiment of the insert of the present disclosure.
FIG. **25** is a bottom view of an alternative embodiment of the insert of the present disclosure.
FIG. **26** is a perspective view of an alternative embodiment of the insert and filter of the present disclosure.
FIG. **27** is another perspective view of an alternative embodiment of the insert and filter of the present disclosure.
FIG. **28** is a perspective view of an embodiment of the present invention having an anatomic design influenced by 3D morphology.
FIG. **29** is a front elevational view of the embodiment having an anatomic design.
FIG. **30** is a rear elevational view of the embodiment having an anatomic design.
FIG. **31** is a right side elevational view of the embodiment having an anatomic design.
FIG. **32** is a left side elevational view of the embodiment having an anatomic design.
FIG. **33** is a top plan view of the embodiment having an anatomic design.
FIG. **34** is a bottom plan view of the embodiment having an anatomic design.
FIG. **35** is a perspective view of the embodiment having an anatomic design being positioned within a nostril and having a bridge.
FIG. **36** is a perspective view of an alternative embodiment having an anatomic design
being positioned within nostrils. wherein the nasal insert is connected a subsequent nasal insert by the bridge.
FIG. **37** is a perspective view of an alternative embodiment having an anatomic design,
wherein a filter is connected to the nasal insert.
FIG. **38** is a perspective view of another alternative embodiment having an anatomic design, wherein an additional leg is attached to the hub.
FIG. **39** is a right side view of the alternative embodiment having an anatomic design and an additional leg.
FIG. **40** is a bottom plan view of the alternative embodiment having an anatomic design and an additional leg.
FIG. **41** is a perspective view of the yet another alternative embodiment having an anatomic design, wherein the arched legs spiral.
FIG. **42** is a front view of the alternative embodiment having an anatomic design,
wherein the arched legs spiral.
FIG. **43** is a top plan view of the alternative embodiment having an anatomic design, wherein the arched legs spiral.

### DETAIL DESCRIPTIONS OF THE INVENTION

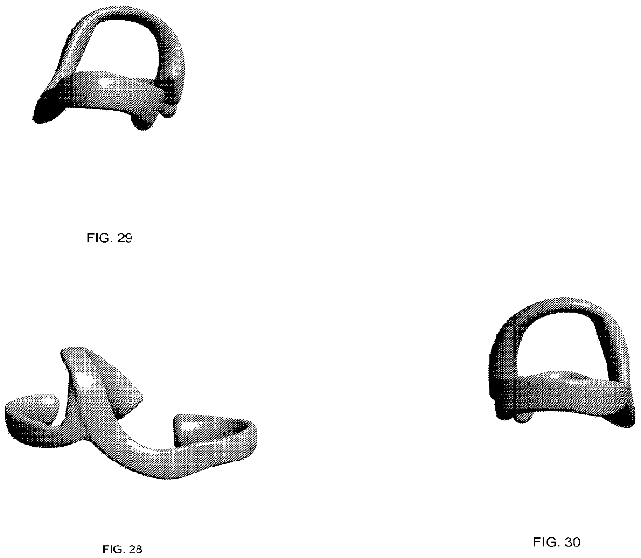

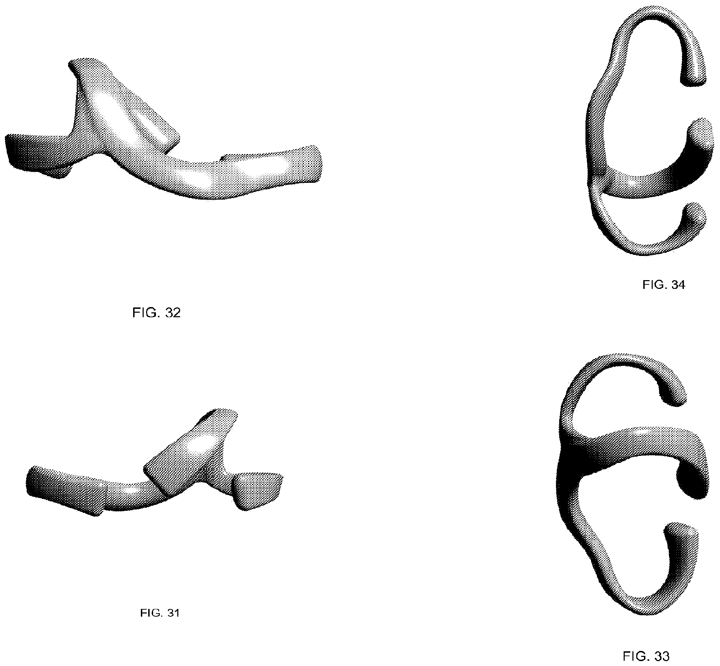

All illustrations of the drawings in Figures 28-36 are for the purpose of describing selected versions of the present invention and are not intended to limit the scope of the present invention.

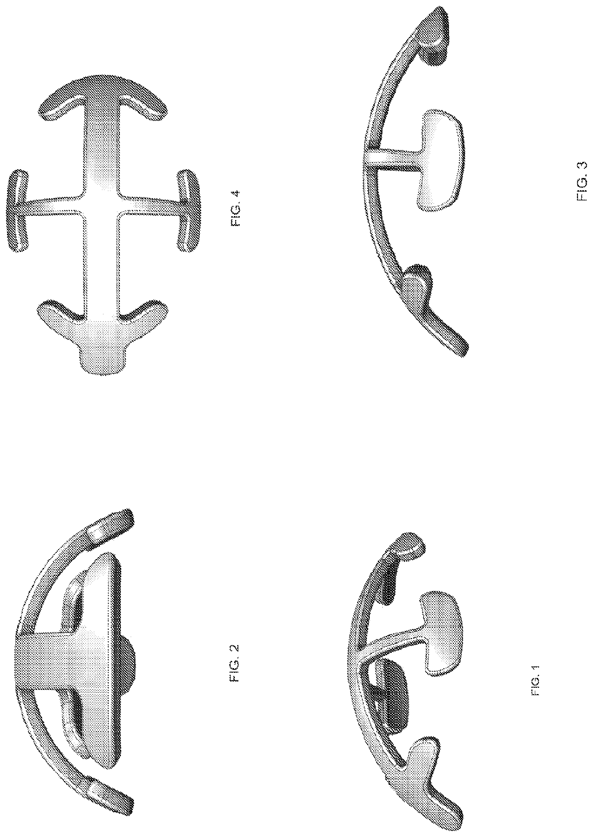

The present invention is a nasal apparatus as described in the claims which is placed in the nostril in order to provide several respiratory benefits. An insert of the present invention comprises a hub and a plurality of arched legs, with the plurality of arched legs being connected around the hub. The plurality of arched legs, in combination with the hub, give the present invention an elliptical dome-shaped appearance. Each of the plurality of arched legs comprises a free end. When inserted into a nostril, the free end of each of the plurality of arched legs presses against the interior surface. A resulting opposing force pushes the connected hub in the opposite direction, causing the nostril to expand due to the forces of the plurality of arched legs and the hub. This configuration and resulting nostril expansion is a core aspect of the present invention, as the increased airway enhances breathing and mitigates related issues such as snoring. The insert, with and without filter, is illustrated in FIG. **1** - FIG. **17****.**

Greater range of nostril size variation within a Type set, for example set I & II, is achieved by incorporating the plurality of arched legs which allow flexibility in key anatomic locations. The length of the each of the plurality of arched legs and the nature of the open profile design allow the device to conform to the individual users nostril. The profile cross section of each leg (generally oval shaped) is designed to allow bending along its thin dimension, while maintaining a relatively larger area of contact in its long dimension. The larger area of contact, thin bending profile, and long beam length reduce the pressure applied by the device when inserted into a relatively smaller nostril.

The free end of each of the plurality of arched legs comprises a foot. The foot is preferably arc shaped in a way that forms a radial perimeter to the hub, as shown in FIG. **1** - FIG. **4****.** The foot provides a greater surface area which can disperse pressure across with the nostril interior and thus provide a more comfortable experience for a user. Preferably, also in the interest of ergonomics, the edge of each foot is smooth and rounded.

In the preferred embodiment the plurality of arched legs comprises a first leg, a second leg, a third leg, and a fourth leg each of which are flexible bars that are radially positioned around the hub. The legs are equally spaced around the hub such that each that the free end of any given leg is separated by an imaginary 90 degree arc of an imaginary circle, the center of which is positioned on the hub and the perimeter of which intersects each free end.

The configuration of an alternative embodiment herein disclosed is designed to orient one pair of legs along an axis running from the apex of the nose to the back of the nose and a second pair of legs along a perpendicular medial axis. The first leg and the third leg are positioned opposite each other around the hub, forming the first pair of legs which is oriented into the nostril beginning at the apex of the nose. The second leg and the fourth leg are similarly positioned opposite each other around the hub, forming the second pair of legs which is oriented along the medial axis. The first leg and second leg are coplanar to their respectively paired third leg and fourth leg. Conversely, the first pair of legs is situated on a plane which is perpendicular to a plane upon which the second pair of legs is situated.

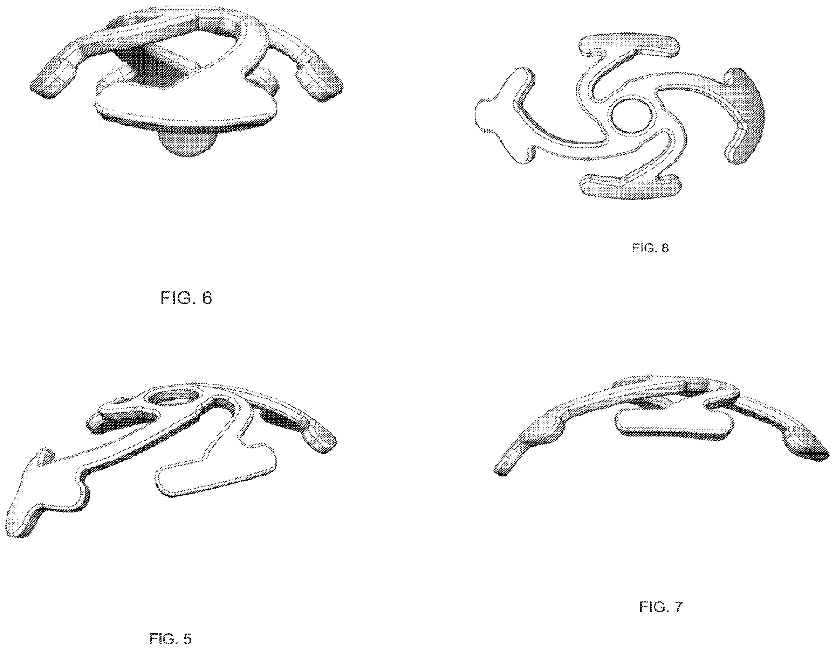

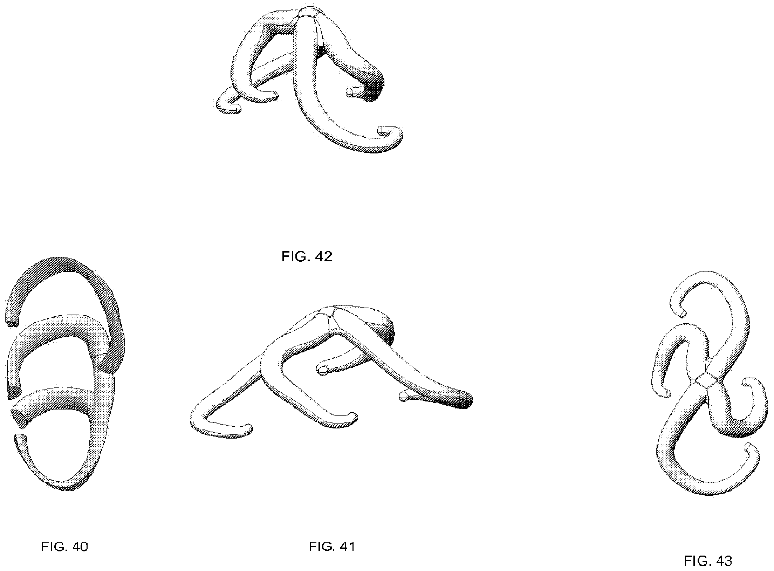

The geometric shape of the legs themselves can vary with different embodiments, examples of which are provided in FIG. **1** - FIG. **4** and FIG. **5** - FIG. **8****.** While FIG. **1** - FIG. **4** depict a regular embodiment with a simple-cross based pattern of legs (seen as such when viewed along the axis of the hub), FIG. **5** - FIG. **8** and FIG. **41** - FIG. **43** show how a spiral pattern of legs can be used instead. It is noted that in both embodiments the free ends of the legs are still positioned in a circular pattern with equal arcs separating each free end; this essentially maintains the orientations of each pair of legs as described with the preferred embodiment.

The spiral embodiment comprises a hole traversing through the central of the hub, as clearly illustrated in FIG. **8****.** This spiral embodiment has several advantages and disadvantages compared to the cross based pattern. The primary advantages of the spiral embodiment are that the spiral embodiment is easier to place and that the central hole allows for greater airflow. However, the spiral embodiment is also bulkier and takes up more room than an embodiment utilizing the cross based pattern.

The free end of the first leg, which is positioned adjacent to the nostril opening when in use, comprises a tab. This tab, an elongated section of the foot, is perpendicular to the arc of the corresponding foot and oriented along the same apex-rear axis as the first pair of legs. This tab provides an easily accessed handle that allows a user to pull the alternative embodiment of the present disclosure out of the nostril for removal. When the alternative embodiment of the disclosure is properly inserted into a nostril, the tab is similarly positioned within the nostril and out of sight of other persons.

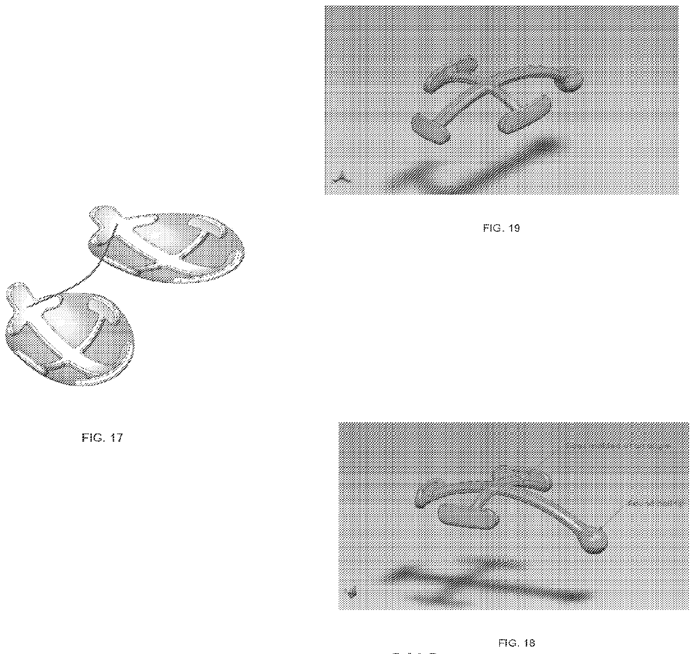

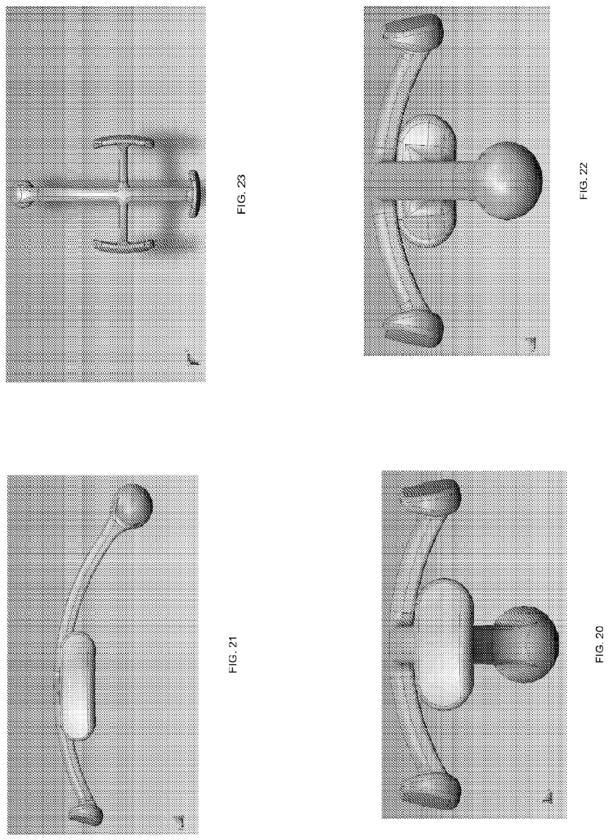

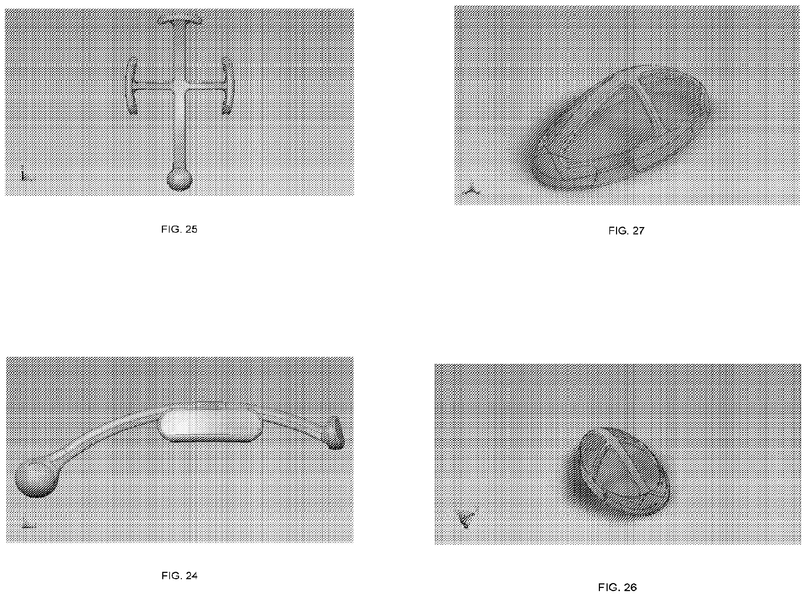

In another embodiment of the present disclosure, the foot of the second leg, the third leg, and the fourth leg is molded at an angle. As a result, each foot comprises a lateral face which presses against the nasal walls (rather than the nasal floor as with the preferred embodiment). The tab of the first leg replaces the foot, being configured as a round ball tip. This alternative embodiment provides an alternative configuration that improves ergonomics and usability while still being able to provide the same functions and benefits of the first described embodiment. Due to these improvements, the alternative embodiment is considered more suitable for mass production and retail. Illustrations of this alternative embodiment are provided in FIG. **18** - FIG. **27****.**

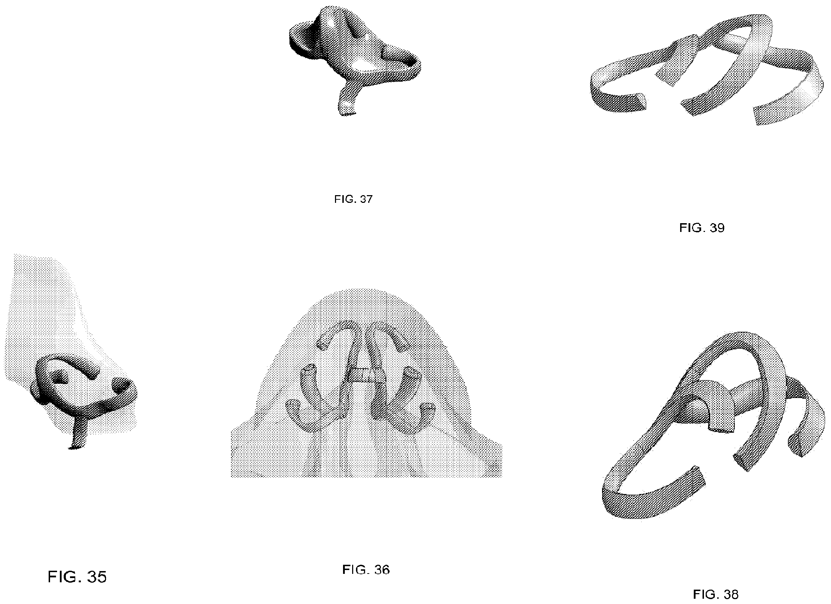

In reference to FIG. **28-34**, showing embodiments of the present invention, the profile of the present invention is anatomically influenced, and has three legs. They merge on the medial side of the nostril and form the largest contact area at the septal cartilage, which is relatively planar. One leg extends in the anterior direction, following the structure of the majar alar cartilage. A second leg extends in the posterior direction and follows the curvature of the alar fibrofatty tisse structure. A third leg extends superior and arcs around to an inferior orientation, terminating near the posterior portion of the majar alar cartilage. The three legs serve to dilate the nasal cavity by acting on these structures. The flexibility of the device within and between each leg is primarily in the medial/lateral direction, and secondarily in the anterior/posterior direction. In reference to FIG. **38-40**, a fourth leg may also be included for additional locational stability.

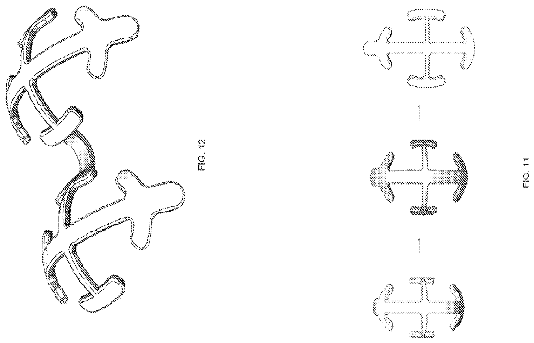

The nasal apparatus as heretofore described is provided for a single nostril. Preferably, the present invention is distributed in pairs, with an insert provided for each nostril. These pairs can be completely independent from each other such that they may easily be inserted or removed individually. Alternatively, a left nostril insert and a right nostril insert can be connected by a bridge which is connected to each nostril at the tab of the first leg. The bridge is a thin strip of material which rests against the columella of the nose; this positioning minimizes exposure of the bridge and places it such that it is mostly obscured and unassuming without directed observation. The intention is for the bridge to be essentially hidden such that it may be worn throughout the day without attracted undesired attention to a user. The visibility of the flexible bridge can be further reduced or eliminated in various ways. One non-limiting example is using a translucent or transparent material for the bridge construction. Other ways of hiding or obscuring the bridge, when included, can be utilized in other embodiments. To provide more flexibility to a user of the present invention, the bridge can be removably attached to each insert; this allows a user the choice of using the bridge for better structure or removing the bridge for better aesthetics or comfort. A user can even alter between using the bridge and not as their personal preferences or situational circumstances change. Depictions of the bridge are provided in FIG. **12**, FIG. **17**, and FIG. **35****-36.**

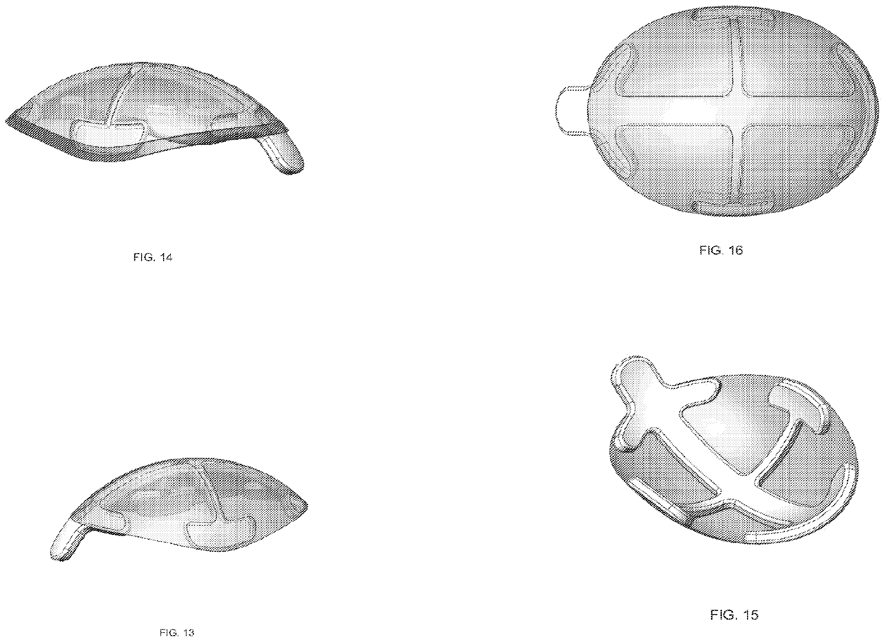

In addition to the insert, the present invention may further comprise a separate attachable filter piece. The filter can be readily incorporated into the design via insert-molding, adhesive, or welding (thermal or ultrasonic). If adapted for user with a filter, the insert further comprises a rim that intersects the free end of each leg and is positioned around the center of the insert. The rim is preferably elliptical in shape to match the three-dimensional shape of the insert. This rim acts as a rim-mounting surface, preferably made of a silicone material to help retain an attached filter. Expanding upon the improved respiratory benefits, the attachable filter is provided to mitigate allergens, dust particles, and other foreign bodies which might agitate or disrupt a person's nasal airways and breathing. The filter is shaped to fit over the insert, sharing the same general dome shape. The apex of the filter is positioned atop the center of the hub while the perimeter of the filter is positioned around the plurality of legs, adjacent to each free end of the legs. Preferably, the perimeter of the filter comprises an adhesive coating to help secure the filter to the rim of the insert. Several views of the filter are provided in FIG. **13** - FIG. **17** and FIG. **37****.** The filter is attached to the insert through molding, in which an injection mold of the insert body is created around the insert. The insert body is permeated in several sections to allow for a secure attachment of the filter via adhesive insert molding. The attachment process can utilize a variety of methods, such as ultrasonic welding or hot joining, to complete the attachment of the filter to the insert.

To use the present invention, a user places a nasal insert in each nostril such that the first leg and associated tab are positioned adjacent to the nostril opening, the hub of the insert is positioned adjacent to the roof of the nostril, and the feet of the insert are positioned on the floor of the nostril. Once placed inside the nostril, the insert presses against the interior nostril surfaces such that the airways is expanded. To conform the insert to the nostril, a user can press on the exterior of their nose, causing the insert to take a shape matching the nostril interior. The applied force acting upon the insert (via the nostril wall) causes the insert to flatten and compress, enabled by its elastic and flexible construction. This allows the insert to better adapt to the interior of the nose while still increasing expanding the airway. Furthermore, in an embodiment which includes the filter, the combination of the filter and insert create a seal with respect to the nasal cavity, which prevents the flow of air from circumventing the filter.

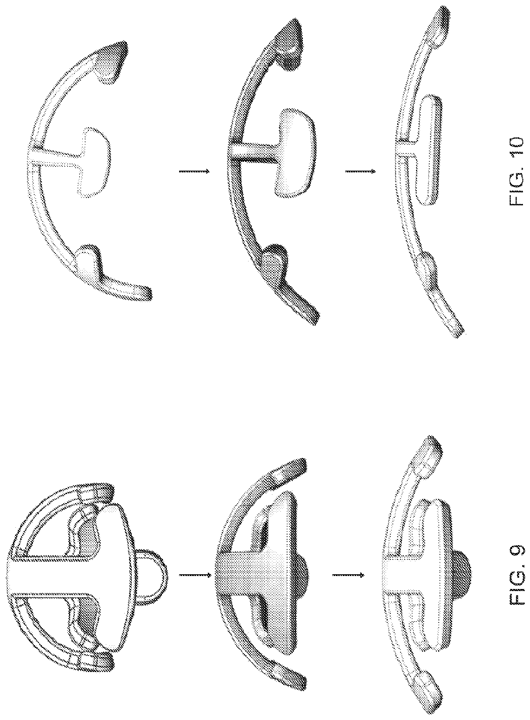

The elastic and flexible construction of the insert are especially beneficial as they allow for a better range of fits with a single model size. There are a wide variety of individual characteristics which mean that what one person considers a good or comfortable fit may be poorly suited to another. Such differences are not limited across people, as a person's features are not perfectly symmetrical and their individual left and right nostrils can vary in size. Correct sizing is important with regards to nostrils, as devices which are too big are very uncomfortable while devices which are too small are not secure and less effective. The present invention overcomes these limitations, as the insert compresses and flexes to conform to a user's individual nostril. As a result, the present invention is adaptable to individual variations in nostril size and shape, providing a "one size fits all" solution. The compression of the present invention is depicted from several views in FIG. **9** - FIG. **11****.**

The compressive forces applied to the insert by the nose and the plurality of legs help keep the inserts inside the nostril during regular everyday activities. The present invention is, minus the bridge when used, internally secured and not visible in normal situations. When a user wishes to remove the inserts and filters, such as for removal or replacement, they can simply apply pressure to the tip of the nose. Pressure should be applied in an upwards direction and in a direction opposite of the nostril the insert is being removed from. Thus, to remove an insert from the left nostril pressure is applied upwards and the tip of the nose is moved to the right, while to remove an insert from the right nostril the tip of the nose is instead moved to the left. This results in further compression of the insert, which assumes a flatter shape. As the insert flattens, the tab which is normally positioned adjacent to the nostril opening is moved out of the nostril, providing an easily gripped handle. This exposed tab makes it simple for a user to grasp and remove the insert.

The filter and the inserts are preferably made in dark colors which do not contrast the interior of the nose and thus are effectively invisible to outside viewpoints. It is noted that when the present invention both utilizes a bridge and is made from a single mold, the bride and the inserts must be made from the same material; in this scenario the bridge and inserts are made of a transparent or translucent material to better hide the bridge. This is comparison to a bridgeless embodiment in which the inserts are made in a dark color. The filter itself can vary in several aspects, the most notable of which is the fineness of the filtering medium. In a preferred embodiment the filter prevents passage of particles that large than 0.1 microns and smaller than 100 microns in size. This range has been selected as filters which are finer than a tenth of a micron may inhibit airflow and breathing, while a filter that allows passage to particles larger than one hundred microns may prove ineffective against a number of potential allergens and other irritating foreign bodies.

Ideally, the filter prevents passage of harmful or irritating particles including but not limited to bacteria, viruses, pathogens, allergens, dust, pollen, pollutants, toxins, carcinogens, and airborne particles capable of causing disease.

Although the invention has been explained in relation to its preferred embodiment, it is to be understood that many other possible modifications and variations can be made without departing from the scope of the invention.

## Claims

1. A nasal apparatus comprising:
a nasal insert of elastic and flexible construction, the nasal insert comprises a hub, a plurality of arched legs wherein:
a) each of the plurality of arched legs comprises a proximal end and a free end;
b) the proximal end and the free end are opposite ends of the same leg;
c) the free end of each of the plurality of arched legs comprises a foot;
d) the proximal end of each of the plurality of arched legs being adjacently connected to the hub; wherein the free ends are disjointed from the hub;
e) the plurality of arched legs is positioned around the hub;
f) the nasal insert is flexible, and applies pressure to the inner nasal surface of the nostril when the nasal insert is positioned within the nostril, thereby holding the insert within the nostril(s); and wherein
g) the plurality of arched legs allows flexibility in key anatomic locations, the profile cross section of the leg being configured to allow bending along its thin dimension, while maintaining a relatively large area of contact in its long dimension,
h) the device being adaptable to different nostril sizes;
wherein the apparatus comprises a first leg, a second leg, and a third leg, which 3 legs merge on the medial side of the nostril, forming the largest surface area at the septal cartilage, when the apparatus is inserted into the nostril(s); and wherein, when the apparatus is inserted into the nostril(s):
i) the first leg is configured to extend in an anterior direction, following the structure of the majar alar cartilage of the nostril;
ii) the second leg is configured to extend in a posterior direction, following the curvature of an alar fibrofatty tissue structure; and
iii) the third leg is configured to extend in a superior direction, and then to arc around to an inferior orientation, terminating near a posterior portion of the majar alar cartilage.

2. The nasal apparatus of claim 1, comprising a pair of nasal apparatus conjoined by a bridge.

3. The conjoined nasal apparatus of claim 2, wherein the bridge is removably attached to each nasal apparatus of the pair.

4. The nasal apparatus of claim 1 comprising:
a) a filter being a separate attachable filter piece;
b) wherein the filter is shaped to fit over the insert, sharing the same general dome shape.

5. The nasal-apparatus of claim 4, wherein the filter is attached to the nasal apparatus through a molding process, where an injection mold of the insert body is created around the nasal apparatus.

6. The nasal apparatus of claim 5 wherein:
a) the nasal apparatus comprises a rim;
b) the filter comprises an adhesive coating;
c) the rim intersects the free end of each of the plurality of arched legs; and
d) the filter is secured to the rim via an adhesive coating.

7. The nasal apparatus of claim 2, wherein the bridge connecting the conjoined pair of nasal apparatus is formed of a translucent or transparent material.

8. The nasal apparatus of claim 4, wherein the filter is incorporated into the nasal apparatus via insert-molding, adhesive, or thermal or ultrasonic welding.

9. The nasal apparatus of claim 6, wherein the rim is elliptical in shape to match the three-dimensional shape of the insert.

10. The nasal apparatus of claim 6, wherein the perimeter of the filter comprises an adhesive coating to secure the filter to the rim.

11. The nasal apparatus of claim 6 wherein the rim acts as a rim-mounting surface, the rim being made of a silicone material to help retain an attached filter.

12. The nasal apparatus of claim 6, wherein the apex of the filter is positioned atop the center of the hub while the perimeter of the filter is positioned around the plurality of legs, adjacent to each free end of the legs.

13. The nasal apparatus of claim 1, wherein the foot of the second leg, third leg and fourth leg is molded at an angle; and optionally, wherein the edge of each foot is smooth and rounded.

## Patentansprüche

1. Nasale Vorrichtung, umfassend:
einen Naseneinsatz von elastischer und flexibler Konstruktion, wobei der Naseneinsatz eine Nabe, eine Vielzahl von gewölbten Schenkeln umfasst, wobei:
a) jeder von der Vielzahl von gewölbten Schenkeln ein proximales Ende und ein freies Ende umfasst;
b) das proximale Ende und das freie Ende gegenüberliegende Enden desselben Schenkels sind;
c) das freie Ende von jedem von der Vielzahl von gewölbten Schenkeln einen Fuß umfasst;
d) wobei das proximale Ende von jedem der Vielzahl von gewölbten Schenkeln angrenzend mit der Nabe verbunden ist;
wobei die freien Enden von der Nabe getrennt sind;
e) die Vielzahl von gewölbten Schenkeln um die Nabe herum positioniert ist;
f) der Naseneinsatz flexibel ist und Druck auf die innere Nasenfläche des Nasenlochs ausübt, wenn der Naseneinsatz innerhalb des Nasenlochs positioniert ist, wodurch der Einsatz innerhalb des Nasenlochs (der Nasenlöcher) gehalten wird; und wobei
g) die Vielzahl von gewölbten Schenkeln Flexibilität an wichtigen anatomischen Stellen ermöglicht, wobei der Profilquerschnitt des Schenkels dazu konfiguriert ist, ein Biegen entlang seiner dünnen Abmessung zu ermöglichen, während eine relativ große Kontaktfläche in seiner langen Abmessung erhalten bleibt,
h) wobei die Vorrichtung an verschiedene Nasenlochgrößen anpassbar ist;
wobei die Vorrichtung einen ersten Schenkel, einen zweiten Schenkel und einen dritten Schenkel umfasst, und die 3 Schenkel auf der medialen Seite des Nasenlochs zusammenlaufen und die größte Oberfläche am Nasenscheidewandknorpel bilden, wenn die Vorrichtung in das Nasenloch eingeführt wird, und wobei, wenn die Vorrichtung in das Nasenloch (die Nasenlöcher) eingeführt wird:
i) der erste Schenkel dazu konfiguriert ist, sich in einer anterioren Richtung zu erstrecken und der Struktur des großen Flügelknorpels des Nasenlochs zu folgen;
ii) der zweite Schenkel dazu konfiguriert ist, sich in einer posterioren Richtung zu erstrecken und der Krümmung einer fettig-fibrösen Flügelgewebestruktur zu folgen; und
iii) der dritte Schenkel dazu konfiguriert ist, sich in einer superioren Richtung zu erstrecken und sich dann in einer inferioren Ausrichtung umzubiegen und in der Nähe eines posterioren Abschnitts des großen Flügelknorpels zu enden.

2. Nasale Vorrichtung nach Anspruch 1, umfassend ein Paar von nasalen Vorrichtungen, die durch eine Brücke zusammengefügt sind.

3. Zusammengefügte nasale Vorrichtung nach Anspruch 2, wobei die Brücke abnehmbar an jeder nasalen Vorrichtung des Paares befestigt ist.

4. Nasale Vorrichtung nach Anspruch 1, umfassend:
a) einen Filter, der ein separates, aufsteckbares Filterstück ist;
b) wobei der Filter so geformt ist, dass er über den Einsatz passt und die gleiche allgemeine Kuppelform aufweist.

5. Nasale Vorrichtung nach Anspruch 4, wobei der Filter durch ein Formverfahren an der nasalen Vorrichtung befestigt wird, wobei eine Spritzgießform des Einsatzkörpers um die nasale Vorrichtung herum erzeugt wird.

6. Nasale Vorrichtung nach Anspruch 5, wobei:
a) die nasale Vorrichtung einen Rand umfasst;
b) der Filter eine Klebstoffbeschichtung umfasst;
c) der Rand das freie Ende eines jeden von der Vielzahl von gewölbten Schenkeln schneidet; und
d) der Filter an dem Rand über eine Klebstoffbeschichtung befestigt ist.

7. Nasale Vorrichtung nach Anspruch 2, wobei die Brücke, die das zusammengefügte Paar von nasalen Vorrichtungen verbindet, aus einem transluzenten oder transparenten Material gebildet ist.

8. Nasale Vorrichtung nach Anspruch 4, wobei der Filter in die nasale Vorrichtung durch Umspritzen, Kleben, Thermooder Ultraschallschweißen eingearbeitet wird.

9. Nasale Vorrichtung nach Anspruch 6, wobei der Rand elliptisch geformt ist, um mit der dreidimensionalen Form des Einsatzes übereinzustimmen.

10. Nasale Vorrichtung nach Anspruch 6, wobei der Umfang des Filters eine Klebstoffbeschichtung umfasst, um den Filter an dem Rand zu befestigen.

11. Nasale Vorrichtung nach Anspruch 6, wobei der Rand als eine Randmontagefläche wirkt, wobei der Rand aus einem Silikonmaterial hergestellt ist, um das Festhalten eines angebrachten Filters zu erleichtern.

12. Nasale Vorrichtung nach Anspruch 6, wobei der Scheitelpunkt des Filters auf der Mitte der Nabe positioniert ist, während der Umfang des Filters um die Vielzahl von Schenkeln herum benachbart zu jedem freien Ende der Schenkel positioniert ist.

13. Nasale Vorrichtung nach Anspruch 1, wobei der Fuß des zweiten Schenkels, des dritten Schenkels und des vierten Schenkels in einem Winkel geformt ist; und wobei optional die Kante von jedem Fuß glatt und abgerundet ist.

## Revendications

1. Appareil nasal comprenant :
un insert nasal de structure élastique et souple, l'insert nasal comprend un élément central, une pluralité de pattes arquées, dans lequel :
a) chacune de la pluralité de pattes arquées comprend une extrémité proximale et une extrémité libre ;
b) l'extrémité proximale et l'extrémité libre sont des extrémités opposées de la même patte ;
c) l'extrémité libre de chacune de la pluralité de pattes arquées comprend un pied ;
d) l'extrémité proximale de chacune de la pluralité de pattes arquées étant reliée de manière adjacente à l'élément central ; les extrémités libres étant disjointes de l'élément central ;
e) la pluralité de pattes arquées sont positionnées autour de l'élément central ;
f) l'insert nasal est souple et applique une pression sur la surface nasale interne de la narine lorsque l'insert nasal est positionné à l'intérieur de la narine, maintenant ainsi l'insert à l'intérieur de la ou des narines ; et
g) la pluralité de pattes arquées permettent une flexibilité à des emplacements anatomiques clés, la section transversale de profil de la patte étant configurée pour permettre une courbure le long de sa dimension mince, tout en maintenant une surface de contact relativement grande dans sa dimension longue,
h) le dispositif étant adaptable à différentes tailles de narine ;
l'appareil comprenant une première patte, une deuxième patte et une troisième patte, lesquelles 3 pattes fusionnant sur le côté médian de la narine, formant la surface la plus grande au niveau du cartilage septal, lorsque l'appareil est introduit dans la ou les narines ; et, lorsque l'appareil est introduit dans la ou les narines :
i) la première patte est configurée pour s'étendre dans une direction antérieure, suivant la structure du cartilage latéral principal de la narine ;
ii) la deuxième patte est configurée pour s'étendre dans une direction postérieure, suivant la courbure d'une structure de tissu fibreux gras latéral ; et
iii) la troisième patte est configurée pour s'étendre dans une direction supérieure, puis s'arquer autour d'une orientation inférieure, se terminant près d'une partie postérieure du cartilage latéral principal.

2. Appareil nasal selon la revendication 1, comprenant une paire d'appareils nasaux conjoints par un pont.

3. Appareil nasal conjoint selon la revendication 2, dans lequel le pont est attaché de manière amovible à chaque appareil nasal de la paire.

4. Appareil nasal selon la revendication 1, comprenant :
a) un filtre qui est un élément filtrant attachable distinct ;
b) le filtre étant formé pour s'ajuster sur l'insert, partageant la même forme générale de dôme.

5. Appareil nasal selon la revendication 4, dans lequel le filtre est attaché à l'appareil nasal par un procédé de moulage, un moule d'injection du corps d'insert étant créé autour de l'appareil nasal.

6. Appareil nasal selon la revendication 5, dans lequel :
a) l'appareil nasal comprend un rebord
b) le filtre comprend un revêtement adhésif ;
c) le rebord coupe l'extrémité libre de chacune de la pluralité de pattes arquées ; et
d) le filtre est fixé au rebord par l'intermédiaire d'un revêtement adhésif.

7. Appareil nasal selon la revendication 2, dans lequel le pont reliant la paire conjointe d'appareils nasaux est formé d'un matériau translucide ou transparent.

8. Appareil nasal selon la revendication 4, dans lequel le filtre est incorporé dans l'appareil nasal par moulage d'insert, adhésif ou soudage thermique ou soudage par ultrasons.

9. Appareil nasal selon la revendication 6, dans lequel le rebord est de forme elliptique pour correspondre à la forme tridimensionnelle de l'insert.

10. Appareil nasal selon la revendication 6, dans lequel le périmètre du filtre comprend un revêtement adhésif pour fixer le filtre au rebord.

11. Appareil nasal selon la revendication 6, dans lequel le rebord sert de surface de montage de rebord, le rebord étant fait d'un matériau de silicone pour aider à retenir un filtre attaché.

12. Appareil nasal selon la revendication 6, dans lequel le sommet du filtre est positionné au-dessus du centre de l'élément central tandis que le périmètre du filtre est positionné autour de la pluralité de pattes, de manière adjacente à chaque extrémité libre des pattes.

13. Appareil nasal selon la revendication 1, dans lequel le pied de la deuxième patte, de la troisième patte et de la quatrième patte est moulé à un angle ; et, facultativement, le bord de chaque pied est lisse et arrondi.
